Europäisches Patentamt

European Patent Office

Office européen des brevets

(19)

(11) **EP 0 351 605 B1**

(12) **EUROPÄISCHE PATENTSCHRIFT**

(45) Veröffentlichungstag und Bekanntmachung des Hinweises auf die Patenterteilung:
**10.04.1996 Patentblatt 1996/15**

(51) Int Cl.[6]: **G01N 33/84**

(21) Anmeldenummer: **89111898.6**

(22) Anmeldetag: **30.06.1989**

(54) **Quantitative Bestimmung von Phosphor**

Quantitative determination of phosphorus

Détermination quantitative de phosphore

(84) Benannte Vertragsstaaten:
**AT BE CH DE ES FR GB IT LI NL SE**

(30) Priorität: **18.07.1988 US 220772**

(43) Veröffentlichungstag der Anmeldung:
**24.01.1990 Patentblatt 1990/04**

(73) Patentinhaber: **F. HOFFMANN-LA ROCHE AG
CH-4002 Basel (CH)**

(72) Erfinder:
• **Kaufman, Richard Allen
Belleville, N.J. 07109 (US)**
• **Rosenfeld, Henry Joseph
Florham Park, N.J. 07932 (US)**
• **Zaunczkowski, Denise
Wallington, N.J. 07057 (US)**

(74) Vertreter: **Kloter, Rudolf et al
Postfach 3255
Grenzacherstrasse 124
CH-4002 Basel (CH)**

(56) Entgegenhaltungen:
**FR-A- 2 165 651**

• **ANAL. BIOCHEM., Band 88, Nr. 2, 1978, Academic PressInc.; S. TSUYOSHI et al., Seiten 347-356#**
• **CHEMICAL ABSTRACTS, Band 98, Nr. 13, 28. März 1983, Columbus, OH (US); M.A. MUNOZ et al., Seite 312, Nr. 103808x#**
• **BIOCHEMICA ET BIOPHYSICA ACTA, Band 320, 1973, Elsevier Scientific Publishing Co., Amsterdam (NL); A. ATKINSON et al., Seiten 195-204#**

**Beschreibung**

Die Erfindung betrifft ein photometrisches Einreagensverfahren zum quantitativen Bestimmen von anorganischem Phosphor gemäss Oberbegriff von Anspruch 1 sowie eine Zusammensetzung für denselben Zweck.

Es wird hierbei von einem Stand der Technik nach dem Patent FR-2 165 651 ausgegangen.

Mit diesem vorbekannten Verfahren sind mehrere Nachteile verbunden. Erstens verlangt dieses, dass die erste Absorptionsmessung innerhalb von 2 Sekunden nach Mischen von Reagenz und Probe der Körperflüssigkeit durchgeführt wird. Eine derartige Beschränkung bewirkt, dass dieses Verfahren äusserst umständlich durchzuführen ist. Zweitens ist darauf hinzuweisen, dass in der Reaktionsgeschwindigkeit zwischen Protein (z.B. Serum) und Proben auf wässriger Basis ein Unterschied besteht, welcher zur Folge hat, dass Proben auf Proteinbasis schneller reagieren als Proben auf wässriger Basis. Dieses Verfahren erfordert daher eine separate Kalibrierung für Phosphorbestimmungen in wässrigen Proben von Körperflüssigkeiten, wie Urin, und in Körperflüssigkeiten auf Proteinbasis, wie Serum.

Aufgabe der vorliegenden Erfindung ist es, diese Nachteile, nämlich die überaus schnelle Handlungsnotwendigkeit zur Durchführung der ersten Absorptionsbestimmung und die verschiedene Kalibrierung für wässrige und Protein enthaltende Körperflüssigkeiten, zu beseitigen.

Die Lösung dieser Aufgabe erfolgt durch die Massnahmen gemäss des kennzeichnenden Teils des Anspruchs 1, wobei Weiterbildungen des erfindungsgemässen Verfahrens durch die Merkmale der Ansprüche 2 bis 8 gekennzeichnet sind.

Eine Zusammensetzung, die für das erfindungsgemässe Verfahren zur Anwendung gelangen kann, ist gekennzeichnet durch die Ansprüche 9 bis 13.

Nach einem ersten Aspekt der vorliegenden Erfindung wird ein Verfahren zur quantitativen Bestimmung von Phosphor in Körperflüssigkeiten bereitgestellt, welches eine photometrische Messung des Molybdatbildungsreaktion beinhaltet, welch letztere sehr langsam vor sich geht, so dass die erste der zwei Absorptionsmessungen bis zu 10 Sekunden nach Mischen des Reagens mit der Körperflüssigkeit vorgenommen werden kann.

Nach einem weiteren Aspekt der vorliegenden Erfindung wird ein Verfahren zur quantitativen Bestimmung von Phosphor in Körperflüssigkeiten bereitgestellt, bei welchem die gleiche Kalibration sowohl für Proben auf wässriger Basis, wie Urin, oder Proben auf Proteinbasis, wie Serum, verwendet werden kann. Dieses Verfahren erlaubt die gleichzeitige Durchführung von wässrigen und Serumproben für die quantitative Bestimmung von Phosphor.

Die quantitative Bestimmung von Phosphor in Körperflüssigkeiten ist wichtig in der Diagnose von verschiedenen Krankheiten, wie Urämie und chronischen Nierenerkrankungen, bei denen eine Phosphorretention auftritt.

Gemäss vorliegender Erfindung wird nur die Menge der sogenannten anorganischen Phosphate in Körperflüssigkeiten bestimmt Wenn immer in der Beschreibung auf Konzentrationen oder Mengen an Phosphor Bezug genommen wird, bedeutet dies Phosphor, das in diesen anorganischen Phosphaten vorkommt.

Die Bedeutung von Veränderungen in organischem Phosphor, wie in Phospholipiden, Phosphatestern und Nukleotidphosphaten, ist klinisch weniger relevant.

Die Zusammensetzung und die Herstellung des Reagens wird später beschrieben. Die angegebenen Konzentrationen beziehen sich auf die Konzentrationen der einzelnen Bestandteile des vollständig formulierten Reagens.

Es bedeutet M Mol pro Liter; mM Millimol pro Liter und % $\frac{W}{V}$ Gramm pro 100 ml Lösung. Demgemäss sind 0,01 g in 100 ml Lösung 0,01% $\frac{W}{V}$. Aehnlich sind 0,5 g in 100 ml Lösung 0,5% $\frac{W}{V}$.

Das oberflächenaktive Mittel des Reagens muss so sein, dass Serum und wässrige Körperflüssigkeiten mit denselben Phosphorkonzentrationen dieselben Absorptionswerte ergaben. Das oberflächenaktive Mittel muss auch die Ausfällung von Proteinen in den zu testenden Körperflüssigkeiten verhindern. Ein Beispiel eines solchen oberflächenaktiven Mittels ist der nicht-ionische Polyoxyäthylen (23) Laryläther, welches die chemische Formel $C_{12}H_{25}$ $(OCH_2CH_2)_{23}OH$ aufweist. Ein besonders bevorzugtes oberflächenaktives Mittel ist der nicht-ionische Polyoxyäthylen (20) Oleyläther, welches die chemische Formel $C_{18}H_{35}(OCH_2CH_2)_{20}OH$ aufweist.

Beispiele von schwermetallchelierenden Mitteln sind Zitronensäure oder bevorzugt N-Hydroxyäthyläthylendiamintriessigsäure.

Das Antioxidans des Reagens kann ein Antioxidans sein, welches die Oxidation des oberflächenaktiven Mittels verhindert. Ein besonders bevorzugtes Antioxidans ist butyliertes Hydroxyanisol.

Als starke Säuren für das Reagens kommen die meisten Säuren in Betracht, die in wässriger Lösung fast vollständig ionisieren. Beispiele solcher starken Säuren sind Alkyl- und aromatische Sulfonsäuren und bevorzugt Alkylsulfonsäuren, wie Methansulfonsäure und Aethansulfonsäure, sowie Arylsulfonsäuren, wie Phenylsulfonsäure und Naphthalinsulfonsäure. Benzoesäuren, wie Trinitrobenzoesäure, können ebenfalls verwendet werden. Andere starke Säuren sind Salzsäure, Trifluoressigsäure, Fluorborsäure, Hydrojodsäure, schweflige Säure, Jodsäure, Perjodsäure, Selensäure, Oxalsäure, Maleinsäure, Dichloressigsäure und Cyclopropan-1,1- dicarbonsäure. Eine speziell bevorzugte starke Säure für das Reagens gemäss der Erfindung ist Schwefelsäure.

Ein typisches Reagens wurde wie folgt hergestellt:

Es wurde eine Lösung durch Lösen von 7 g Brij® 99, 0,01 g butyliertes Hydroxyanisol und 0,15 g N-Hydroxyäthyläthylendiamintriessigsäure in ungefähr 500 ml entionisiertem Wasser hergestellt. Zu dieser Lösung wurden 41,7 ml konzentrierte Schwefelsäure zugegeben. Dann wurden 2,55 g Ammoniummolybdat darin gelöst und schliesslich wurden 100 µl Antischaummittel Colloid 1010 zugegeben. Zusätzlich wurde entionisiertes Wasser zugegeben, um ein Gesamtvolumen von 1 Liter des Reagens zu erhalten.

Das oberflächenaktive Mittel im obigen Beispiel, nämlich Brij® 99 ist Polyoxyäthylen (20) Oleyläther mit der chemischen Formel $C_{18}H_{35}(OCH_2CH_2)_{20}OH$, dem Molekulargewicht 1146,56 und dem Schmelzpunkt 25-30°C.

Colloid 1010 wird von Colloids Inc., 394 Frelinghuysen Avenue, Newark, N.J. 07114 verkauft. Colloid 1010 ist ein Schaumkontrollmittel auf Silikonbasis mit den folgenden typischen Eigenschaften:

| Erscheinen: | Opaque, stabil flüssig |
|---|---|
| Farbe: | weiss |
| Typ: | stabile Emulsion |
| Nicht flüchtiger Rückstand bei 105°C: | 15% |
| Spezifisches Gewicht bei 25°C: | 1 |
| Silikongehalt: | 10% |

Um Schaumprobleme zu vermeiden, wird in einer bevorzugten Ausführungsform des Reagens ein Antischaummittel zugegeben.

Ein Reagens kann mit Komponenten in den folgenden Konzentrationen formuliert werden. Die Konzentrationen für die einzelnen Bestandteile beziehen sich auf das vollständig formulierte Reagens.

Zu einer Lösung von entionisiertem Wasser enthaltend ungefähr 0,01% $\frac{W}{V}$ bis ungefähr 5% $\frac{W}{V}$ an oberflächenaktivem Mittel, ungefähr 0,001% $\frac{W}{V}$ bis ungefähr 0,5% $\frac{W}{V}$ N-Hydroxyäthyläthylendiamintriessigsäure und ungefähr 0,0001% bis ungefährt 0,01% $\frac{W}{V}$ butyliertes Hydroxyanisol wird Schwefelsäure in einer Konzentration von ungefähr 0,675 M bis ungefähr 1,25 M zugegeben. Zu dieser Lösung wird Ammoniummolybdat in einer Konzentration von ungefähr 1,75 mM bis ungefähr 6,25 mM zugegeben und dann wird gegebenenfalls ein Antischaummittel im Bereich von ungefähr 0,001% $\frac{W}{V}$ bis ungefähr 0,1% $\frac{W}{V}$ zugegeben.

Die Vorteile des Reagens sind die folgenden: Das Reagens ist eine einzige Reagenzienmischung im Vergleich zu den zwei Reagenzienmischungen gemäss dem Stand der Technik. Demgemäss ist keine Vortestung erforderlich.

Weiterhin ist die Reaktion zwischen Probe und Reagens langsamer als beim Stand der Technik, was bedeutet, dass bis zu 10 Sek. verstreichen können bevor die erste Absorptionsmessung vorgenommen wird. Demgemäss ist die Phosphorbestimmung gemäss vorliegender Erfindung bequemer als die vorbekannten Methoden zur Durchführung von Phosphorbestimmungen, wie z.B. diejenige gemäss U.S. Patent 3,795,484, bei dem die erste Absorptionsmessung innerhalb von 2 Sekunden nach Bildung des Gemisches von Reagens und Probe vorgenommen werden muss. Die erste Absorptionsmessung wird vorzugsweise nach 4,5 Sek. nach Mischen von Reagens und Probe vorgenommen.

Schliesslich besteht ein wesentlicher Vorteil der vorliegenden Erfindung gegenüber dem Stand der Technik darin, dass wässrige und Serumproben gemeinsam bestimmt werden können unter Verwendung einer einzigen Kalibrierung, welche entweder auf einem wässrigen oder einem Serumstandard basiert. Dies ist dadurch bedingt, dass das Reagens gemäss der Erfindung so gestaltet ist, dass die Aenderung in der Absorption von wässrigen oder Serumproben mit gleicher Phosphorkonzentrationen gleich ist, obwohl die Reaktionsgeschwindigkeiten für wässrige und Serumproben gleicher Konzentrationen leicht unterschiedlich sind.

Das Reagens kann mit einem nicht-zentrifugalen oder einem zentrifugalen analytischen Photometer, bevorzugt mit dem COBAS™ BIO®, FARA® oder MIRA® durch geführt werden.

Nach dem Verfahren gemäss vorliegender Erfindung wird der Phosphorgehalt in Körperflüssigkeiten quantitativ bestimmt durch Bilden einer Mischung von Körperflüssigkeit, entionisiertem Wasser als Diluens und von Reagens. Das Volumenverhältnis von Körperflüssigkeit zu Diluens und Reagens kann von ungefähr 1:125 bis 1:30 variieren und ist bevorzugt ungefähr 1:50. Es erfolgt dann eine erste Absorptionsmessung bei 340 Nanometern. Diese erste Absorptionsmessung kann bis spätestens 10 Sekunden nach Bildung der Mischung durchgeführt werden. Diese Absorptionsmessung wird mit einem nicht-zentrifugalen oder bevorzugt mit einem zentrifugalen Analytikphotometer durchgeführt.

Eine zweite Absorptionsmessung wird innerhalb von 10 Min. nach Bildung des Gemisches durchgeführt. Da die Reaktion der Körperflüssigkeit mit dem Reagens relativ gering ist, kann die erste Absorptionsmessung als Probenleerwert verwendet werden.

Um die Menge des Phosphors in der Körperflüssigkeit zu bestimmen, wird die Differenz zwischen den zwei Absorptionsmessungen gegen die Absorptionen von Flüssigkeiten mit bekannten Konzentrationen von Phosphor verglichen.

In den nachfolgenden Tests, welche die Effizienz der vorliegenden Erfindung zeigen, werden 5 µl Probe, 50 µl Diluens (entionisiertes Wasser) und 200 µl Reagens parallel in die entsprechenden Behälter des Küvettenrotors COBAS BIO® gegeben. Die Reaktion wird dann bei 340 Nanometern überwacht.

Eine erste Absorptionsmessung wird 4,5 Sek. nach Mischen durchgeführt. Da zu diesem Zeitpunkt lediglich eine geringe Menge der Reaktion stattgefunden hat, kann diese Absorptionsmessung als Probenleerwert dienen. Eine zweite Messung wird dann 3 Min. nach Mischen durchgeführt, zu einer Zeit, da die Reaktion praktisch beendet ist. Die Konzentration des Phosphors wird durch Multiplizieren der Differenz zwischen der letzten und der ersten Absorption mit einem Kalibrationsfaktor berechnet, welch letzterer von Standards von bekannten Phosphorkonzentrationen berechnet wird.

Eine Absorptionsmessung, die früh in der Reaktion durchgeführt wird, liefert einen Probenleerwert, der es erlaubt, lipämische, ikterische und hämolysierte Proben exakt und ohne Interferenzen durchzuführen.

<u>Tests, die zeigen, dass das Reagens gemäss vorliegender Erfindung die Fähigkeit hat, den Probenleerwert zu bestimmen</u>

Die folgenden Materialien werden in diesem Test verwendet:

1. Wässrige Standards enthaltend anorganischen Phosphor (3,6 und 12 mg/dL Phosphor)

2. Normal Humanserum - (hitzeinaktiviert, filtriert, enthaltend 0,1 % Azid) - aufgestockt mit $KH_2PO_4$ (Kaliumphosphat) liefert Proben von 1,5, 4, 6, 8, 10, 12, 14, 16 und 18 mg/dL Phosphor.

3. Lipämische und hämolysierte Proben.

4. Das Reagens für den anorganischen Phosphor wird unten beschrieben (Zweireagenziensystem mit Probenleerwert).

5. Einreagenssystem gemäss der vorliegenden Erfindung.

Die lipämischen und hämolysierten Patientenproben werden zuerst unter Verwendung des Zweireagenziensystems, welches bekanntlich einen Probenleerwert liefert, bestimmt. Nach dem Zweireagenziensystem wird die Probe zuerst mit Reagens, welche 0,36 mol/l Schwefelsäure enthält, gemischt und es wird ein Probenleerwert bei 340 Nanometern gemessen. Ein zweites Reagens, welches 5,4 mmol/l Ammoniummolybdat enthält, wird dann zugegeben, worauf nach 15 Sek. eine zweite Absorptionsmessung bei 340 Nanometern durchgeführt wird. Die erste Absorption wird von der zweiten subtrahiert um die Nettoabsorptionsdifferenz, die den Phosphorgehalt der Probe wiederspiegelt, zu erhalten. Dieselben Proben werden dann unter Verwendung des Einreagenziensystems getestet.

<u>Ergebnisse:</u>

Tabelle I zeigt vergleichbare Resultate welche von lipämischen und hämolysierten Proben gemäss dem obenerwähnten Zweireagenziensystem und dem Einreagenssystem der vorliegenden Erfindung erhalten wurden. Sie zeigen, dass das Einreagenssystem bzgl. Genauigkeit mit einer vorbekannten Bestimmung für Phosphor, die eine Messung des Probenleerwertes beinhaltet, vergleichbar ist. Tabelle I zeigt somit die Fähigkeit des Reagens der Erfindung zur Erfassung des Probenleerwertes.

Tabelle I

|  | Probe | 2 Reagenzien | 1 Reagens |
|---|---|---|---|
| lipämisch | 1 | 2,8 mg/dl | 2,3 mg/dl |
|  | 2 | 4,5 | 4,4 |
|  | 3 | 4,1 | 3,9 |
|  | 4 | 3,7 | 3,6 |
|  | 5 | 17,2 | 16,9 |

Fortsetzung der Tabelle auf der nächsten Seite

Tabelle I   (fortgesetzt)

|  | Probe | 2 Reagenzien | 1 Reagens |
|---|---|---|---|
| hämolysiert | 1 | 14,7 | 14,3 |
|  | 2 | 3,5 | 3,0 |
|  | 3 | 2,8 | 2,9 |
|  | 4 | 3,9 | 3,8 |

<u>Tests, die zeigen, dass Proben auf wässriger und Serumbasis mit einem wässrigen Standard durchgeführt werden können</u>

Die folgenden Materialien wurden in diesem Test verwendet:

1. Wässrige Standards enthalten anorganischen Phosphor (3, 6 und 12 mg/dl Phosphor).

2. Wässrige Proben - destilliertes Wasser aufgestockt mit Kaliumphosphat um Proben von 1,5, 4, 6, 8, 10, 12, 14, 16 und 18 mg/dl Phosphor zu erhalten.

3. Serumproben - normales humanes Serum (hitzeinaktiviert, filtriert, enthaltend 0,1% Azid) aufgestockt mit Kaliumphosphat um Proben von 1,5, 4, 6, 8, 10, 12, 14, 16 und 18 mg/dl Phosphor zu erhalten.

4. Das Reagens für anorganischen Phosphor wie oben beschrieben (Zweireagenziensystem mit Probenleerwert).

5. Einreagenssystem gemäss der vorliegenden Erfindung.

<u>Ergebnisse:</u>

Wässrige und Serumproben von gleicher Phosphorkonzentration werden auf dem COBAS BIO® mit dem oben beschriebenen Zweireagenziensystem und dem Einreagenssystem gemäss der Erfindung bestimmt, wobei lediglich ein wässriger Standard zur Kalibrierung der Instrumente verwendet wurde.

Tabelle II zeigt die Phosphorkonzentration für die wässrigen Standards, die zur Kalibration verwendet wurden und die Phosphorkonzentrationen der wässrigen und Serumproben, die gegen diese Kalibrationskurve bestimmt wurden. Neben jeder Phosphorkonzentration ist die Aenderung der Absorption bei 340 Nanometern für die erste Ablesung nach 4,5 Sek. und der zweiten Ablesung nach 3 Min. angegeben. Es ist zu beachten, dass die Aenderung in der Absorption für die wässrigen und Serumproben mit gleicher oder ähnlicher Phosphorkonzentration gleich oder proportional sind, was zeigt, dass wässrige und Serumproben mit dem Einreagenssystem mit einem einzigen wässrigen Standard für die Kalibrierung durchgeführt werden können und zwar unäbhängig davon, dass eine Differenz in der Reaktionsgeschwindigkeit zwischen wässrigen und Serumproben besteht.

## Tabelle II

| Wässriger Standard | | | Wässrige Probe | | | Serumprobe | | |
|---|---|---|---|---|---|---|---|---|
| Phosphorkonzentration | | ΔA | Phosphorkonzentration | | ΔA | Phosphorkonzentration | | ΔA |
| | | | 1,6 mg/dl | | 0,14 | 1,6 mg/dl | | 0,14 |
| 3 mg/dl | | 0,27 | 3,7 | | 0,32 | 3,7 | | 0,33 |
| 6 | | 0,53 | 5,8 | | 0,51 | 5,8 | | 0,51 |
| | | | 8,3 | | 0,73 | 7,8 | | 0,69 |
| | | | 10,2 | | 0,90 | 9,9 | | 0,87 |
| 12 | | 1,03 | 11,7 | | 1,03 | 11,9 | | 1,05 |
| | | | 13,6 | | 1,20 | 13,8 | | 1,21 |
| | | | 15,3 | | 1,35 | 15,7 | | 1,38 |
| | | | 17,3 | | 1,52 | 17,6 | | 1,56 |

$$\Delta A = A_{3Min.} - A_{4,5\ Sek.}$$

Tests, die zeigen, dass wässrige und Serumproben mit entweder wässrigen oder Serumstandards durchgeführt werden können

Die folgenden Materialien werden in diesem Test verwendet:

1. Wässrige Standards enthaltend Phosphor (3, 6 und 12 mg/dl Phosphor).

2. Humanserumproben von bekanntem Phosphorgehalt.

3. Normal Humanserum (hitzeinaktiviert, filtriert, 0,1% Azid enthaltend) aufgestockt mit Kaliumphosphat, um Proben von 1,5, 4, 6, 8, 10, 12 und 14 mg/dl Phosphat zu erhalten.

4. Urinproben 1:10 mit entionisiertem Wasser verdünnt.

5. Normale und lipämische Humanserumproben.

Bei der Durchführung dieses Tests werden die Proben zuerst mit dem oben beschriebenen Zweireagenziensystem unter Verwendung wässriger Standards für die Kalibration bestimmt. Die gleichen Proben werden mit dem Einreagenssystem bestimmt, zuerst unter Kalibration eines wässrigen Standards und dann unter Kalibration eines Serumstandards.
Die Tabelle III zeigt, dass die Resultate dieser drei verschiedenen Bestimmungen sehr gut miteinander korrelieren und zwar unabhängig davon, ob die Probe Normalserum, lipämisches Serum oder Urin war. Es ist zu beachten, dass die Kalibrationsfaktoren für die wässrigen oder die Serumstandards im wesentlichen dieselben waren.

Ergebnisse:

Tabelle III

| | | 2 Reagenzien Wässriger Standard | | 1 Reagens Wässriger Standard | 1 Reagens Serum-Standard |
|---|---|---|---|---|---|
| Normal-Serum | 1 | | 1,1 mg/dl | 1,2 mg/dl | 1,2 mg/dl |
| | 2 | | 3,8 | 3,9 | 3,9 |
| | 3 | | 5,9 | 5,9 | 6,0 |
| | 4 | | 8,2 | 8,1 | 8,0 |
| | 5 | | 10,2 | 10,2 | 10,3 |
| | 6 | | 12,8 | 12,5 | 12,3 |
| | 7 | | 14,5 | 14,3 | 14,4 |
| Urin | 1 | | 1,1 | 1,1 | 1,1 |
| | 2 | | 0,8 | 0,9 | 0,9 |
| | 3 | | 2,4 | 2,5 | 2,5 |
| | 4 | | 2,0 | 2,1 | 2,1 |
| | 5 | | 4,4 | 4,5 | 4,5 |
| | 6 | | 6,0 | 6,1 | 6,2 |
| Normal-serumproben | 1 | | 4,6 | 4,5 | 4,6 |
| | 2 | | 4,0 | 4,1 | 4,1 |
| | 3 | | 3,8 | 3,9 | 3,9 |
| Lipämische Serumproben | 1 | | 2,9 | 2,5 | 2,5 |
| | 2 | | 4,6 | 4,8 | 4,8 |
| | 3 | | 3,6 | 3,5 | 3,5 |
| | 4 | | 5,1 | 3,9 | 4,1 |
| Kalibrationsfaktor | | | 9,9 | 11,3 | 11,4 |

**Patentansprüche**

**Patentansprüche für folgende Vertragsstaaten : AT, BE, CH, DE, FR, GB, IT, LI, NL, SE**

1. Photometrisches Einreagensverfahren zum quantitativen Bestimmen von anorganischem Phosphor in einer wässrigen oder Protein enthaltenden Körperflüssigkeit, bei dem die Körperflüssigkeit mit einer Ammoniummolybdatlösung und einem oberflächenaktiven Mittel gemischt wird, eine erste Absorptionsmessung kurz nach dem Mischen und eine zweite Absorbtionsmessung innerhalb von 10 Minuten nach dem Mischen erfolgt und der Phosphorgehalt durch Vergleich der Absorptionsdifferenz mit mindestens einer unter gleichen Bedingungen ermittelten Absorptionsdifferenz von einer eine bekannte Menge Phosphor enthaltenden Flüssigkeit bestimmt wird, dadurch gekennzeichnet, dass die Körperflüssigkeit gleichzeitig mit der Ammoniummolybdatlösung, dem oberflächenaktiven Mittel, einer starken Säure, einem Schwermetallchelierungsmittel, einem Antioxidans und entionisiertem Wasser, gegebenenfalls noch mit einem Antischaumnüttel, gemischt wird und die erste Absorptionsmessung nach 2 Sekunden durchgeführt werden kann, jedoch bis spätestens 10 Sekunden nach Bildung der Mischung durchgeführt werden muss.

2. Verfahren nach Anspruch 1, dadurch gekennzeichnet, dass die Körperflüssigkeit mit einer Zusammensetzung, welche oberflächenaktives Mittel im Bereich von 0,01% $\frac{W}{V}$ bis 5% $\frac{W}{V}$, starke Säure im Bereich von 0,675 bis 1,25 M, Ammoniummolybdat im Bereich von 1,75 mM bis 6,25 mM, Schwermetallchelierungsmittel, Antioxidans und entionisiertes Wasser enthält, gemischt wird.

3. Verfahren nach Anspruch 1 oder 2, dadurch gekennzeichnet, dass das oberflächenaktive Mittel Polyoxyäthylen (20) Oleyläther der chemischen Formel $C_{18}H_{35}(OCH_2CH_2)_{20}OH$ ist.

4. Verfahren nach einem der Ansprüche 1 bis 3, dadurch gekennzeichnet, dass die starke Säure Schwefelsäure ist.

5. Verfahren nach einem der Ansprüche 1 bis 4, dadurch gekennzeichnet, dass butyliertes Hydroxyanisol als Antioxidans und N-Hydroxyäthyläthylendiamintriessigsäure als Schwermetallchelierendes Mittel verwendet wird.

6. Verfahren nach Anspruch 5, dadurch gekennzeichnet, dass butyliertes Hydroxyanisol im Bereich von 0,0001% $\frac{W}{V}$ bis 0,01% $\frac{W}{V}$ und N-Hydroxyäthyläthylendiamintriessigsäure im Bereich von 0,001% $\frac{W}{V}$ bis 0,5% $\frac{W}{V}$ verwendet wird.

7. Verfahren nach einem der Ansprüche 1 bis 6, dadurch gekennzeichnet, dass 5 µl Körperflüssigkeit, 50 µl entionisiertes Wasser mit 200 µl einer Zusammensetzung, welche ungefähr 7 g pro Liter Polyoxyäthylen (20) Oleyläther der chemischen Formel $C_{18}H_{35}(OCH_2CH_2)_{20}OH$, ungefähr 41,7 ml pro Liter konzentrierte Schwefelsäure, ungefähr 2,55 g pro Liter Ammoniummolybdat, ungefähr 1,5 g pro Liter N-Hydroxyäthyläthylendiamintriessigsäure, ungefähr 0,01 g pro Liter butyliertes Hydroxyanisol, ungefähr 100 µl pro Liter Antischaummittel und als Rest entionsiertes Wasser enthält, gemischt wird.

8. Verfahren nach einem der Ansprüche 1 bis 7, dadurch gekennzeichnet, dass die Absorptionsmessungen bei 340 nm durchgeführt werden.

9. Zusammensetzung zur quantitativen photometrischen Bestimmung von anorganischem Phosphor in Körperflüssigkeiten, dadurch gekennzeichnet, dass sie ein oberflächenaktives Mittel im Bereich von 0,01% $\frac{W}{V}$ bis 5% $\frac{W}{V}$, eine starke Säure im Bereich von 0,675 M bis 1,25 M, Ammoniummolybdat im Bereich von 1,75 mM bis 6,25 mM, ein Schwermetallchelierungsmittel, ein Antioxidans und entionsiertes Wasser enthält.

10. Zusammensetzung nach Anspruch 9, dadurch gekennzeichnet, dass das oberflächenaktive Mittel Polyoxyäthylen (20) Oleyläther der chemischen Formel $C_{18}H_{35}(OCH_2CH_2)_{20}OH$ ist.

11. Zusammensetzung nach Anspruch 9, dadurch gekennzeichnet, dass die starke Säure Schwefelsäure ist.

12. Zusammensetzung nach einem der Ansprüche 9 bis 11, dadurch gekennzeichnet, dass sie als Schwermetallchelierungsmittel N-Hydroxyäthyläthylendiamintriessigsäure im Bereich von 0,001% $\frac{W}{V}$ bis 0,5% $\frac{W}{V}$ und als Antioxidans butyliertes Hydroxyanisol im Bereich von 0,0001% bis 0,01% $\frac{W}{V}$ enthält.

13. Zusammensetzung nach Anspruch 9, dadurch gekennzeichnet, dass sie ungefähr 7 g pro Liter Polyoxyäthylen (20) Oleyläther der chemischen Formel $C_{18}H_{35}(OCH_2CH_2)_{20}OH$, ungefähr 41,7 ml pro Liter konzentrierte Schwefelsäure, ungefähr 2,55 g pro Liter Ammoniummolybdat, ungefähr 1,5 g pro Liter N-Hydroxyäthyläthylendiamintriessigsäure, ungefähr 0,01 g pro Liter butyliertes Hydroxyanisol, ungefähr 100 µl pro Liter Antischaummittel und als Rest entionisiertes Wasser enthält.


**Patentansprüche für folgenden Vertragsstaat : ES**

1. Photometrisches Einreagensverfahren zum quantitativen Bestimmen von anorganischem Phosphor in einer wässrigen oder Protein enthaltenden Körperflüssigkeit, bei dem die Körperflüssigkeit mit einer Ammoniummolybdatlösung und einem oberflächenaktiven Mittel gemischt wird, eine erste Absorptionsmessung kurz nach dem Mischen und eine zweite Absorbtionsmessung innerhalb von 10 Minuten nach dem Mischen erfolgt und der Phosphorgehalt durch Vergleich der Absorptionsdifferenz mit mindestens einer unter gleichen Bedingungen ermittelten Absorptionsdifferenz von einer eine bekannte Menge Phosphor enthaltenden Flüssigkeit bestimmt wird, dadurch gekennzeichnet, dass die Körperflüssigkeit gleichzeitig mit der Ammoniummolybdatlösung, dem oberflächenaktiven Mittel, einer starken Säure, einem Schwermetallchelierungsmittel, einem Antioxidans und entionisiertem Wasser, gegebenenfalls noch mit einem Antischaummittel, gemischt wird und die erste Absorptionsmessung nach 2 Sekunden durchgeführt werden kann, jedoch bis spätestens 10 Sekunden nach Bildung der Mischung durchgeführt werden muss.

2. Verfahren nach Anspruch 1, dadurch gekennzeichnet, dass die Körperflüssigkeit mit einer Zusammensetzung, welche oberflächenaktives Mittel im Bereich von 0,01% $\frac{W}{V}$ bis 5% $\frac{W}{V}$, starke Säure im Bereich von 0,675 bis 1,25 M, Ammoniummolybdat im Bereich von 1,75 mM bis 6,25 mM, Schwermetallchelierungsmittel, Antioxidans und entionisiertes Wasser enthält, gemischt wird.

3. Verfahren nach Anspruch 1 oder 2, dadurch gekennzeichnet, dass das oberflächenaktive Mittel Polyoxyäthylen (20) Oleyläther der chemischen Formel $C_{18}H_{35}(OCH_2CH_2)_{20}OH$ ist.

4. Verfahren nach einem der Ansprüche 1 bis 3, dadurch gekennzeichnet, dass die starke Säure Schwefelsäure ist.

5. Verfahren nach einem der Ansprüche 1 bis 4, dadurch gekennzeichnet, dass butyliertes Hydroxyanisol als Antioxidans und N-Hydroxyäthyläthylendiamintriessigsäure als Schwermetallchelierendes Mittel verwendet wird.

6. Verfahren nach Anspruch 5, dadurch gekennzeichnet, dass butyliertes Hydroxyanisol im Bereich von 0,0001% $\frac{W}{V}$ bis 0,01 $\frac{W}{V}$ und N-Hydroxyäthyläthylendiamintriessigsäure im Bereich von 0,001% $\frac{W}{V}$ bis 0,5% $\frac{W}{V}$ verwendet wird.

7. Verfahren nach einem der Ansprüche 1 bis 6, dadurch gekennzeichnet, dass 5 µl Körperflüssigkeit, 50 µl entionisiertes Wasser mit 200 µl einer Zusammensetzung, welche ungefähr 7 g pro Liter Polyoxyäthylen (20) Oleyläther der chemischen Formel $C_{18}H_{35}(OCH_2CH_2)_{20}OH$, ungefähr 41,7 ml pro Liter konzentrierte Schwefelsäure, ungefähr 2,55 g pro Liter Ammoniummolybdat, ungefähr 1,5 g pro Liter N-Hydroxyäthyläthylendiamintriessigsäure, ungefähr 0,01 g pro Liter butyliertes Hydroxyanisol, ungefähr 100 µl pro Liter Antischaummittel und als Rest entionsiertes Wasser enthält, gemischt wird.

8. Verfahren nach einem der Ansprüche 1 bis 7, dadurch gekennzeichnet, dass die Absorptionsmessungen bei 340 nm durchgeführt werden.


**Claims**


**Claims for the following Contracting States : AT, BE, CH, DE, FR, GB, IT, LI, NL, SE**

1. A photometric process using a single reagent for the quantitative determination of inorganic phosphorus in an aqueous or protein-containing body fluid, in which an ammonium molybdate solution and a surface active agent are admixed with the body fluid, a first absorbance reading is taken shortly after the mixing and a second absorbance reading is taken within 10 minutes after the mixing and the amount of phosphorus is determined by comparing the absorbance difference with at least one absorbance difference, determined under the same conditions, of a fluid containing a known amount of phosphorus, characterized in that the body fluid is mixed simultaneously with the ammonium molybdate solution, the surface active agent, a strong acid, a heavy metal chelating agent, an antioxidant and deionized water, optionally also with an anti-foaming agent, and the first absorbance reading can be taken after 2 seconds, but must be taken at the latest 10 seconds after formation of the mixture.

2. A process according to claim 1, characterized in that the body fluid is mixed with a composition which contains a surface active agent in the range of 0.01% w/v to about 5% w/v, a strong acid in the range of 0.675 to 1.25M, ammonium molybdate in the range of 1.75 mM to 6.25 mM, a heavy metal chelating compound, an antioxidant and deionized water.

3. A process according to claim 1 or 2, characterized in that the surface active agent is polyoxyethylene (20) oleyl ether of the chemical formula $C_{18}H_{35}(OCH_2CH_2)_{20}OH$.

4. A process according to any one of claims 1 to 3, characterized in that the strong acid is sulphuric acid.

5. A process according to any one of claims 1 to 4, characterized in that butylated hydroxyanisole is used as the

EP 0 351 605 B1

antioxidant and N-hydroxyethylethylenediaminetriacetic acid is used as the heavy metal chelating agent.

6. A process according to claim 5, characterized in that butylated hydroxyanisole is used in the range of 0.0001% w/v to 0.01% w/v and N-hydroxyethylethylenediaminetriacetic acid is used in the range of 0.001% w/v to 0.5% w/v.

7. A process according to any one of claims 1 to 6, characterized by mixing 5 μl of body fluid, 50 μl of deionized water and 200 μl of a composition containing about 7 g per litre of polyoxyethylene (20) oleyl ether of the chemical formula $C_{18}H_{35}(OCH_2CH_2)_{20}OH$, about 41.7 ml per litre of concentrated sulphuric acid, about 2.55 g per litre of ammonium molybdate, about 1.5 g per litre of N-hydroxyethylethylenediaminetriacetic acid, about 0.01 g per litre of butylated hydroxyanisole, about 100 μl of anti-foaming agent and as the remainder deionized water.

8. A process according to any one of claims 1 to 7, characterized in that the absorbance readings are carried out at 340 nm.

9. A composition for the quantitative photometric determination of inorganic phosphorus in body fluids, characterized in that it contains a surface active agent in the range of 0.01% w/v to 5% w/v, a strong acid in the range of 0.675M to 1.25M, ammonium molybdate in the range of 1.75 mM to 6.25 mM, a heavy metal chelating agent, an antioxidant and deionized water.

10. A composition according to claim 9, characterized in that the surface active agent is polyoxyethylene (20) oleyl ether of the chemical formula $C_{18}H_{35}(OCH_2CH_2)_{20}OH$.

11. A composition according to claim 9, characterized in that the strong acid is sulphuric acid.

12. A composition according to any one of claims 9 to 11, characterized in that it contains N-hydroxyethylethylenediaminetriacetic acid as the heavy metal chelating agent in the range of 0.001% w/v to 0.5% w/v and butylated hydroxyanisole as the antioxidant in the range of 0.0001% w/v to 0.01% w/v.

13. A composition according to claim 9, characterized in that it contains about 7 g per litre of polyoxyethylene (20) oleyl ether of the chemical formula $C_{18}H_{35}(OCH_2CH_2)_{20}OH$, about 41.7 ml per litre of concentrated sulphuric acid, about 2.55 g per litre of ammonium molybdate, about 1.5 g per litre of N-hydroxyethylethylenediaminetriacetic acid, about 0.01 g per litre of butylated hydroxyanisole, about 100 μl of anti-foaming agent and as the remainder deionized water.

**Claims for the following Contracting State : ES**

1. A photometric process using a single reagent for the quantitative determination of inorganic phosphorus in an aqueous or protein-containing body fluid, in which an ammonium molybdate solution and a surface active agent are admixed with the body fluid, a first absorbance reading is taken shortly after the mixing and a second absorbance reading is taken within 10 minutes after the mixing and the amount of phosphorus is determined by comparing the absorbance difference with at least one absorbance difference, determined under the same conditions, of a fluid containing a known amount of phosphorus, characterized in that the body fluid is mixed simultaneously with the ammonium molybdate solution, the surface active agent, a strong acid, a heavy metal chelating agent, an antioxidant and deionized water, optionally also with an anti-foaming agent, and the first absorbance reading can be taken after 2 seconds, but must be taken at the latest 10 seconds after formation of the mixture.

2. A process according to claim 1, characterized in that the body fluid is mixed with a composition which contains a surface active agent in the range of 0.01% w/v to about 5% w/v, a strong acid in the range of 0.675 to 1.25M, ammonium molybdate in the range of 1.75 mM to 6.25 mM, a heavy metal chelating compound, an antioxidant and deionized water.

3. A process according to claim 1 or 2, characterized in that the surface active agent is polyoxyethylene (20) oleyl ether of the chemical formula $C_{18}H_{35}(OCH_2CH_2)_{20}OH$.

4. A process according to any one of claims 1 to 3, characterized in that the strong acid is sulphuric acid.

5. A process according to any one of claims 1 to 4, characterized in that butylated hydroxyanisole is used as the antioxidant and N-hydroxyethylethylenediaminetriacetic acid is used as the heavy metal chelating agent.

10

**6.** A process according to claim 5, characterized in that butylated hydroxyanisole is used in the range of 0.0001% w/v to 0.01% w/v and N-hydroxyethylethylenediaminetriacetic acid is used in the range of 0.001% w/v to 0.5% w/v.

**7.** A process according to any one of claims 1 to 6, characterized by mixing 5 µl of body fluid, 50 µl of deionized water and 200 µl of a composition containing about 7 g per litre of polyoxyethylene (20) oleyl ether of the chemical formula $C_{18}H_{35}(OCH_2CH_2)_{20}OH$, about 41.7 ml per litre of concentrated sulphuric acid, about 2.55 g per litre of ammonium molybdate, about 1.5 g per litre of N-hydroxyethylethylenediaminetriacetic acid, about 0.01 g per litre of butylated hydroxyanisole, about 100 µl of anti-foaming agent and as the remainder deionized water.

**8.** A process according to any one of claims 1 to 7, characterized in that the absorbance readings are carried out at 340 nm.

**Revendications**

**Revendications pour les Etats contractants suivants : AT, BE, CH, DE, FR, GB, IT, LI, NL, SE**

**1.** Procédé photométrique à un seul réactif pour la détermination quantitative du phosphore minéral dans un liquide corporel aqueux ou contenant des protéines, dans lequel on mélange le liquide corporel avec une solution de molybdate d'ammonium et un agent tensioactif, on effectue une première mesure de l'absorption peu de temps après le mélange et une seconde mesure de l'absorption en l'espace de 10 minutes après le mélange, et on détermine la teneur en phosphore par comparaison de la différence d'absorption avec au moins une différence d'absorption, déterminée dans les mêmes conditions, d'un liquide contenant une quantité connue de phosphore, caractérisé en ce que le liquide corporel est mélangé simultanément avec la solution de molybdate d'ammonium, l'agent tensioactif, un acide fort, un agent chélateur de métaux lourds, un antioxydant et de l'eau désionisée, éventuellement encore avec un antimousse, et la première mesure de l'absorption peut être effectuée au bout de 2 secondes, mais doit l'être jusqu'au plus tard 10 secondes après la formation du mélange.

**2.** Procédé selon la revendication 1, caractérisé en ce que le liquide corporel est mélangé avec une composition qui contient un agent tensioactif dans la plage allant de 0,01% p/v à 5 % p/v, un acide fort dans la plage allant de 0,675 à 1,25 M, du molybdate d'ammonium dans la plage allant de 1,75 mM à 6,25 mM, un agent chélateur de métaux lourds, un antioxydant et de l'eau désionisée.

**3.** Procédé selon la revendication 1 ou 2, caractérisé en ce que l'agent tensioactif est le polyoxyéthylène (20) oléyléther, de formule chimique $C_{18}H_{35}(OCH_2CH_2)_{20}OH$.

**4.** Procédé selon l'une des revendications 1 à 3, caractérisé en ce que l'acide fort est l'acide sulfurique.

**5.** Procédé selon l'une des revendications 1 à 4, caractérisé en ce que l'on utilise comme antioxydant l'hydroxyanisole butylé et, comme agent chélateur de métaux lourds, l'acide N-hydroxyéthylèthylènediaminetriacétique.

**6.** Procédé selon la revendication 5, caractérisé en ce que l'on utilise l'hydroxyanisole butylé dans la plage allant de 0,0001 % p/v à 0,01 % p/v et l'acide N-hydroxyéthyléthylènediaminetriacétique dans la plage allant de 0,001% p/v à 0,5 % p/v.

**7.** Procédé selon l'une des revendications 1 à 6, caractérisé en ce que l'on mélange 5 µl de liquide corporel, 50 µl d'eau désionisée et 200 µl d'une composition qui contient environ 7 g/l de polyoxyéthylène (20) oléyléther de formule chimique $C_{18}H_{35}(OCH_2CH_2)_{20}OH$, environ 41,7 ml/l d'acide sulfurique concentré, environ 2,55 g/l de molybdate d'ammonium, environ 1,5 g/l d'acide N-hydroxyéthyléthylène-diaminetriacétique, environ 0,01 g/l d'hydroxyanisole butylé, environ 100 µl/l d'antimousse et, comme reste, de l'eau désionisée.

**8.** Procédé selon l'une des revendications 1 à 7, caractérisé en ce que les mesures d'absorption sont effectuées à 340 nm.

**9.** Composition pour la détermination photométrique quantitative du phosphore minéral dans des liquides corporels, caractérisée en ce qu'elle contient un agent tensioactif dans la plage allant de 0,01 % p/v à 5 % p/v, l'acide fort dans la plage allant de 0,675 à 1,25 M, le molybdate d'ammonium dans la plage allant de 1,75 mM à 6,25 mM, un agent chélateur de métaux lourds, un antioxydant et de l'eai désionisée.

10. Composition selon la revendication 9, caractérisée en ce que l'agent tensioactif est le polyoxyéthylène (20) oléyléther, de formule chimique $C_{18}H_{35}(OCH_2CH_2)_{20}OH$.

11. Procédé selon la revendication 9, caractérisé er ce que l'acide fort est l'acide sulfurique.

12. Composition selon l'une des revendications 9 à 11, caractérisée en ce qu'elle contient, en tant qu'agent chélateur de métaux lourds, de l'acide N-hydroxyéthyléthylènediaminetriacétique dans la plage allant de 0,001 % p/v à 0,5 % p/v et, en tant qu'antioxydant, de l'hydroxyanisole butylé, dans la plage allant de 0,0001 % p/v à 0,01 % p/v.

13. Composition selon la revendication 9, caractérisée en ce qu'elle contient environ 7 g/l de polyoxyéthylène (20) oléyléther de formule chimique $C_{18}H_{35}(OCH_5CH_2)_{20}OH$, environ 41,7 ml/l d'acide sulfurique concentré, environ 2,55 g/l de molybdate d'ammonium, environ 1,5 g/l d'acide N-hydroxyéthyléthylènediaminetriacétique, environ 0,01 g/l d'hydroxyanisole butylé, environ 100 µl/l d'antimousse et, comme reste, de l'eau désionisée.

**Revendications pour l'Etat contractant suivant : ES**

1. Procédé photométrique à un seul réactif pour la détermination quantitative du phosphore minéral dans un liquide corporel aqueux ou contenant des protéines, dans lequel on mélange le liquide corporel avec une solution de molybdate d'ammonium et un agent tensioactif, on effectue une première mesure de l'absorption peu de temps après le mélange et une seconde mesure de l'absorption en l'espace de 10 minutes après le mélange, et on détermine la teneur en phosphore par comparaison de la différence d'absorption avec au moins une différence d'absorption, déterminée dans les mêmes conditions, d'un liquide contenant une quantité connue de phosphore, caractérisé en ce que le liquide corporel est mélangé simultanément avec la solution de molybdate d'ammonium, l'agent tensioactif, un acide fort, un agent chélateur de métaux lourds, un antioxydant et de l'eau désionisée, éventuellement encore avec un antimousse, et la première mesure de l'absorption peut être effectuée au bout de 2 secondes, mais doit l'être jusqu'au plus tard 10 secondes après la formation du mélange.

2. Procédé selon la revendication 1, caractérisé en ce que le liquide corporel est mélangé avec une composition qui contient un agent tensioactif dans la plage allant de 0,01% p/v à 5 % p/v, un acide fort dans la plage allant de 0,675 à 1,25 M, du molybdate d'ammonium dans la plage allant de 1,75 mM à 6,25 mM, un agent chélateur de métaux lourds, un antioxydant et de l'eau désionisée.

3. Procédé selon la revendication 1 ou 2, caractérisé en ce que l'agent tensioactif est le polyoxyéthylène (20) oléyléther, de formule chimique $C_{18}H_{35}(OCH_2CH_2)_{20}OH$.

4. Procédé selon l'une des revendications 1 à 3, caractérisé en ce que l'acide fort est l'acide sulfurique.

5. Procédé selon l'une des revendications 1 à 4, caractérisé en ce que l'on utilise comme antioxydant l'hydroxyanisole butylé et, comme agent chélateur de métaux lourds, l'acide N-hydroxyéthylèthylènediaminetriacétique.

6. Procédé selon la revendication 5, caractérisé en ce que l'on utilise l'hydroxyanisole butylé dans la plage allant de 0,0001 % p/v à 0,01 % p/v et l'acide N-hydroxyéthyléthylènediaminetriacétique dans la plage allant de 0,001%p/v à 0,5 % p/v.

7. Procédé selon l'une des revendications 1 à 6, caractérisé en ce que l'on mélange 5 µl de liquide corporel, 50 µl d'eau désionisée et 200 µl d'une composition qui contient environ 7 g/l de polyoxyéthylène (20) oléyléther de formule chimique $C_{18}H_{35}(OCH_2CH_2)_{20}OH$, environ 41,7 ml/l d'acide sulfurique concentré, environ 2,55 g/l de molybdate d'ammonium, environ 1,5 g/l d'acide N-hydroxyéthyléthylène-diaminetriacétique, environ 0,01 g/l d'hydroxyanisole butylé, environ 100 µl/l d'antimousse et, comme reste, de l'eau désionisée.

8. Procédé selon l'une des revendications 1 à 7, caractérisé en ce que les mesures d'absorption sont effectuées à 340 nm.